# EUROPÄISCHE PATENTSCHRIFT

(11) **EP 1 663 942 B1**
(45) Veröffentlichungstag und Bekanntmachung des Hinweises auf die Patenterteilung: **04.04.2007**
(21) Anmeldenummer: 04764542.9
(22) Anmeldetag: 27.08.2004
(51) Int. Cl.: C07C 209/48, C07C 211/27

(54) **VERFAHREN ZUR HERSTELLUNG VON META-XYLYLENDIAMIN (MXDA)**
METHOD FOR PRODUCING META-XYLYLENEDIAMINE (MXDA)
PROCEDE DE PRODUCTION DE META-XYLYLENE DIAMINE (MXDA)

(30) Priorität: 10.09.2003 DE 10341613
(43) Veröffentlichungstag der Anmeldung: 07.06.2006
(73) Patentinhaber: BASF Aktiengesellschaft, 67056 Ludwigshafen (DE)
(72) Erfinder: HUGO, Randolf, 67246 Dirmstein (DE); JOURDAN, Sabine, 68163 Mannheim (DE); WENZ, Kirsten, 68161 Mannheim (DE); PREISS, Thomas, 67256 Weisenheim (DE); WECK, Alexander, 67251 Freinsheim (DE)
(86) Internationale Anmeldenummer: PCT/EP2004/009568
(87) Internationale Veröffentlichungsnummer: WO 2005/028417

(56) Entgegenhaltungen:
- EP-A- 1 193 244
- EP-A- 1 209 146
- EP-A- 1 279 661
- GB-A- 1 164 354
- US-A- 2 970 170

## Beschreibung

Die vorliegende Erfindung betrifft ein Verfahren zur Herstellung von meta-Xylylendiamin umfassend die Schritte
Ammonoxidation von meta-Xylol zu Isophthalodinitril, wobei das dampfförmige Produkt der Ammonoxidationsstufe direkt mit einem flüssigen organischen Lösungsmittel in Kontakt gebracht wird (Quench) und
Hydrierung des Isophthalodinitrils in der erhaltenen Quenchlösung oder -suspension.
Xylylendiamin (Bis(aminomethyl)benzol) XDA ist ein nützlicher Ausgangsstoff, z.B. für die Synthese von Polyamiden, Epoxyhärtern oder als Zwischenstufe zur Herstellung von Isocyanaten.

Die zweistufige Synthese von meta-Xylylendiamin (MXDA) durch Ammonoxidation von meta-Xylol und anschließender Hydrierung des erhaltenen Isophthalodinitrils ist bekannt.

EP-A2-1 113 001 (Mitsubishi Gas Chem. Comp.) beschreibt ein Verfahren zur Herstellung von Nitrilverbindungen durch Ammonoxidation entsprechender carbocyclischer oder heterocyclischer Verbindungen, wobei überschüssiger Ammoniak aus dem Reaktionsprodukt recycliert wird. Beschrieben wird auch das direkte in Kontakt bringen des dampfförmigen Produkts der Ammonoxidationsstufe mit einem flüssigen organischen Lösungsmittel, bei dem es sich insbesondere um aliphatische oder aromatische Kohlenwasserstoffe handelt. (Absätze [0045] und [0046]).

EP-A2-1 193 247 und EP-A1-1 279 661 (beide Mitsubishi Gas Chem. Comp.) betreffen ein Verfahren zur Reinigung von Isophthalodinitril (IPDN) bzw. ein Verfahren zur Herstellung von reinem XDA, in dem das Phthalodinitril durch Ammonoxidation von Xylol synthetisiert wird, wobei das dampfförmige Produkt der Ammonoxidationsstufe direkt mit einem flüssigen organischen Lösungsmittel in Kontakt gebracht wird (Quench). Das organische Lösungsmittel ist ausgewählt aus Alkylbenzole, heterocyclische Verbindungen, aromatische Nitrile und heterocyclische Nitrile und hat einen Siedepunkt, der unter dem von Phthalodinitril liegt (EP-A2-1 193 247: Spalte 4, Absatz [0018] und [0019]; EP-A1-1 279 661: Spalten 4-5, Absatz [0023] und [0024]).

EP-A2-1 193 244 (Mitsubishi Gas Chem. Comp.) beschreibt ein Verfahren zur Herstellung von XDA durch Hydrierung von Phthalodinitril, welches in einer vorherigen Stufe durch Ammonoxidation von Xylol synthetisiert wird, wobei das dampfförmige Produkt der Ammonoxidationsstufe direkt mit einem flüssigen organischen Lösungsmittel in Kontakt gebracht wird (Quench) und die erhaltene Quenchlösung oder -suspension der Hydrierung zugeführt wird.
Bevorzugte organische Lösungsmittel sind C₆-C₁₂ aromatische Kohlenwasserstoffe, wie Xylol und Pseudocumol (Spalte 6, Absatz [0027] und [0028]).

DE-A-21 64169 beschreibt auf Seite 6, letzter Absatz, die Hydrierung von IPDN zu meta-XDA in Gegenwart eines Ni- und/oder Co-Katalysators in Ammoniak als Lösungsmittel.

Fünf parallele BASF-Patentanmeldungen mit jeweils gleichem Anmeldetag betreffen jeweils Verfahren zur Herstellung von XDA.

Der vorliegenden Erfindung lag die Aufgabe zugrunde, ein verbessertes wirtschaftliches Verfahren zur Herstellung von hoch reinem meta-Xylylendiamin mit hoher Ausbeute und Raum-Zeit-Ausbeute (RZA) aufzufinden, welches bei mit Verfahren des Stands der Technik (z.B. EP-A2-1 193 244) vergleichbaren Durchsätzen aufgrund verringerter Stoffströme, insbesondere Lösungsmittelströme, inkl. Rückführströme, verkleinerte Apparate und Maschinen ermöglicht.

Demgemäß wurde ein Verfahren zur Herstellung von meta-Xylylendiamin umfassend die Schritte
Ammonoxidation von meta-Xylol zu Isophthalodinitril, wobei das dampfförmige Produkt der Ammonoxidationsstufe direkt mit einem flüssigen organischen Lösungsmittel in Kontakt gebracht wird (Quench) und
Hydrierung des Isophthalodinitrils in der erhaltenen Quenchlösung oder -suspension gefunden, welches dadurch gekennzeichnet ist, dass es sich bei dem organischen Lösungsmittel um N-Methyl-2-pyrrolidon (NMP) handelt.

Das erfindungsgemäße Verfahren lässt sich wie folgt ausführen:

### Ammonoxidationsstufe:

Die Ammonoxidation von meta-Xylol zum entsprechenden Isophthalodinitril wird im allgemeinen nach dem Fachmann bekannten Verfahren durchgeführt.

Die Ammonoxidation des Methylaromaten wird bevorzugt durchgeführt an einem Multioxidkatalysator mit Ammoniak und einem sauerstoffhaltigen Gas (Sauerstoff oder Luft oder beides) in einem Wirbelschichtreaktor oder einem Rohr(bündel)reaktor.

Die Reaktionstemperatur liegt dabei im allgemeinen bei 300 bis 500°C, bevorzugt bei 330 bis 480°C.

Der Katalysator enthält bevorzugt V, Sb und/oder Cr und setzt sich besonders bevorzugt zusammen aus [V, Sb und Alkalimetallen] oder [V, Cr, Mo und B], jeweils als Vollkatalysator oder auf einem inerten Träger.
Als inerter Träger sind bevorzugt SiO₂, Al₂O₃ oder ein Gemisch der beiden oder Steatit.

Solch eine Verfahrensweise ist z.B. in den BASF-Patentanmeldungen EP-A-767 165 und EP-A-699 476 beschrieben, auf die hiermit ausdrücklich Bezug genommen wird.

Auch die BASF-Patentanmeldungen EP-A-222 249, DE-A-35 40 517 und
DE-A-37 00 710 offenbaren geeignete Ammonoxidationskatalysatoren.

Die Ammonoxidation kann auch gemäß den in den eingangs zitierten Anmeldungen EP-A2-1 113 001, EP-A2-1 193 247, EP-A1-1 279 661 und EP-A2-1 193 244 beschriebenen Verfahren durchgeführt werden.

### Quench:

Der bei der Ammonoxidation produzierte Dampf, enthaltend das Wertprodukt Isophthalodinitril, wird direkt mit dem flüssigen organischen Lösungsmittel N-Methyl-2-pyrrolidon (NMP) in Kontakt gebracht (Quench mit NMP als Quenchflüssigkeit, Quenchmittel).

Das für den Quench verwendete NMP kann auch bereits gelöstes oder suspendiertes Isophthalodinitril enthalten.

Durch die plötzliche Temperaturabsenkung beim in Kontakt bringen des dampfförmigen Isophthalodinitrils mit dem flüssigen Lösungsmittel NMP (Quench) wird die Bildung von unerwünschten Neben- und Zersetzungsprodukten, die zur Qualitätsminderung des Isophthalodinitrils und schließlich des MXDAs führen, verringert.

Das dampfförmige Isophthalodinitril wird durch den Quench direkt in das flüssige Lösungsmittel NMP aufgenommen, wobei eine Lösung und/oder eine Suspension entsteht, welche unmittelbar weiter verarbeitet werden kann.

Als Frischzulauf wird im allgemeinen technisches NMP mit einer Reinheit
> 99 Gew.-%, insbesondere > 99,5 Gew.-%, eingesetzt.
Bevorzugt kann aus dem Verfahren zurückgewonnenes NMP als Quenchflüssigkeit eingesetzt werden. Hier kann die Reinheit der Quenchflüssigkeit auch ≤ 99 Gew.-%, z.B. 90-98 Gew.-%, betragen, insbesondere dann, wenn es sich nicht um verfahrensfremde Substanzen (also u.a. um Wasser, Ammoniak, Benzonitril, Tolunitril, Xylol, o-, m- oder p-Methyl-benzylamin, Benzylamin, Xylylendiamin) als Verunreinigungen handelt.

Die Menge des verwendeten Lösungsmittels NMP ist im allgemeinen so bemessen, dass Lösungen/Suspensionen mit einem Isophthalodinitril-Gehalt von 15 bis 75 Gew.-%, bevorzugt 25 bis 60 Gew.-%, erhalten werden.

Die Einleitung des dampfförmigen Austrags der Ammonoxidation, enthaltend das Isophthalodinitril (IPDN), in das flüssige NMP erfolgt in einem Quenchapparat, z.B. bevorzugt in einem Fallfilmkondensator (Dünnschicht-, Rieselfilm- oder Fallstromkondensator), in einem Düsenapparat oder in einer Kolonne. Dabei kann das dampfförmige Isophthalodinitril im Gleich- oder im Gegenstrom mit dem flüssigen Lösungsmittel geführt werden. Bei Gleichstromführung wird das dampfförmige Isophthalodinitril von oben in den Quenchapparat eingeleitet. Vorteilhaft ist die tangentiale Zufuhr des flüssigen Lösungsmittels am Kopf des Fallfilmkondensators oder die Zufuhr des flüssigen Lösungsmittels durch eine oder mehrere Düsen um eine vollständige Benetzung der Innenwand des Quenchapparates zu erreichen.

Im Falle einer Quenchkolonne wird das Gas aus der Ammonoxidation am Kolonnensumpf aufgegeben und das Lösungsmittel am Kopf zugeführt. Der Quenchapparat kann zur Vergrößerung der zur Kondensation verfügbaren Oberfläche mit Einbauten wie Böden, geordneten Packungen oder ungeordneten Schüttungen ausgerüstet sein. Das NMP für den Quench kann im einmaligen Durchlauf oder als Kreislaufmedium eingesetzt werden.

Vorteilhafterweise wird ein Teil der Quenchlösung oder -suspension im Kreis gefahren. Mittels eines im Kreislauf eingebauten Wärmeüberträgers wird die Quenchlösung oder -suspension gekühlt.

Dabei werden die Temperatur des Kreislaufmediums und der Kreislaufmengenstrom so eingestellt und aufeinander abgestimmt, dass die gewünschte Temperatur im Quenchaustritt erreicht wird. Die Temperatur des Kreislaufmediums wird umso niedriger gewählt, je kleiner der Mengenstrom des Kreislaufmediums ist und umgekehrt, wobei Löslichkeiten und Schmelzpunkte sowie die hydraulischen Belastungsgrenzen des Quenchapparates zu berücksichtigen sind.

Der Mengenstrom des frisch zulaufenden NMPs ist von der Quenchtemperatur abhängig, da mit höher werdender Temperatur mehr Lösungsmittel im dampfförmigen Quenchaustrag enthalten ist. Er wird so eingestellt, dass die gewünschte Konzentration der IPDN-Lösung oder -suspension erhalten wird. Da die Löslichkeit von IPDN in NMP mit zunehmender Temperatur ansteigt, kann mit zunehmender Quenchaustrittstemperatur eine höhere IPDN-Konzentration im NMP gefahren werden.

Das Kreislaufmedium wird gemeinsam mit dem frischen Lösungsmittel oder davon getrennt an geeigneter Stelle des Quenchapparats zugefahren.
Im Falle einer im Gegenstrom betriebenen Quenchkolonne wird das frische NMP am Kopf und das Kreislaufmedium weiter unten, etwa in Kolonnenmitte zugefahren.

Im allgemeinen wird durch Temperierung des eingesetzten NMPs und/oder des Kreislaufmediums die Temperatur des flüssigen Quenchaustrags auf 40 bis 180°C, bevorzugt 50 bis 120°C, insbesondere 80 bis 120°C, eingestellt.

Der Siedepunkt von Isophthalodinitril liegt im Bereich von 1 bis 100 mbar um ca. 60 Kelvin über dem Siedepunkt von NMP.

Der Absolutdruck beim Quenchen beträgt im allgemeinen 0,5 bis 1,5 bar. Bevorzugt wird bei leichtem Überdruck gefahren.

Xylol, Wasser, NH₃, CO₂, N₂ etc., die im dampfförmigen Austrag der Ammonoxidation in der Regel enthalten sind, werden unter Quenchbedingungen im Quench-Lösungsmittel NMP nur teilweise oder praktisch nicht gelöst und werden aus dem Quench-Apparat überwiegend gasförmig abgetrennt.

Je niedriger die Quenchtemperatur ist, desto höher ist der Anteil von Wasser und tiefer als JPDN siedenden Nebenkomponenten (bei gleichem Druck) (z.B. Benzonitril, Tolunitril) im flüssigen Quenchaustrag.

In einer besonderen Ausgestaltung des erfindungsgemäßen Verfahrens können daher vor der Hydrierung des Isophthalodinitrils aus der erhaltenen Quenchlösung oder - suspension Wasser und gegebenenfalls Produkte mit einem Siedepunkt niedriger als Isophthalodinitril (bei gleichem Druck) (Leichtsieder; z.B. nicht umgesetztes meta-Xylol, Benzonitril, Tolunitril, jeweils als Heteroazeotrop mit Wasser, Wasser, Benzonitril, Tolunitril; Aufzählung mit zunehmenden Siedepunkt (bei gleichem Druck); wie ggf. auch o-, m- oder p-Methyl-benzylamin, Benzylamin, Xylylendiamin, wobei diese Amine aus zurückgeführten Lösungsmittel von der Hydrierstufe stammen) teilweise oder vollständig destillativ abgetrennt werden.

Diese Abtrennung der Leichtsieder erfolgt bevorzugt in einer Destillationskolonne über Kopf.

Bevorzugt wird eine Destillationskolonne verwendet, welche vorzugsweise mit den üblichen Einbauten zur Erhöhung der Trennleistung, wie Böden, geordnete oder ungeordnete Packungen, etc., ausgerüstet ist.

Die Auslegung der Kolonne (insbesondere Zahl der Trennstufen, Zulaufstelle, Rücklaufverhältnis, etc.) kann, abgestimmt auf die jeweilige Zusammensetzung der Lösung oder Suspension, durch den Fachmann nach ihm geläufigen Methoden vorgenommen werden.

Bevorzugt wird unter vermindertem Druck gefahren, um die Sumpftemperatur zu begrenzen.

### Hydrierung:

Die erhaltene Quenchlösung oder -suspension wird anschließend der Hydrierung zugeführt.
Optional kann vorher der Quenchlösung oder -suspension weiteres NMP oder ein weiteres organisches Lösungsmittel, wie z.B. Xylol, Benzylamin, o-, m- oder p-Methyl-benzylamin, Xylylendiamin und Mischungen hiervon, zugegeben werden.

Es ist also ein kennzeichnendes Merkmal des erfindungsgemäßen Verfahrens, dass das als Zwischenstufe erhaltene Isophthalodinitril nicht in Substanz isoliert wird. Das zu hydrierende Isophthalodinitril wird für die Hydrierstufe nicht von dem im Quench-Schritt verwendeten NMP abgetrennt.

Eine Abtrennung von Hochsiedern, also von Produkten mit einem Siedepunkt höher als Phthalodinitril (bei gleichem Druck), aus der Quenchlösung oder -suspension findet im erfindungsgemäßen Verfahren bevorzugt nicht statt.

Für die Hydrierung des Isophthalodinitrils zum entsprechenden meta-Xylylendiamin wird der Lösung oder Suspension besonders bevorzugt Ammoniak, bevorzugt in flüssiger Form, zugefügt. Das Hinzufügen des Ammoniaks kann direkt nach der Quenchstufe oder erst in der Hydrierstufe erfolgen.

Das Gewichtsverhältnis von Dinitril zu Ammoniak beträgt hierbei im Frischzulauf im allgemeinen 1 : 0,15 bis 1 : 15, vorzugsweise 1 : 0,5 bis 1 : 10, besonders bevorzugt 1 : 1 bis 1 : 5.

Für die Hydrierung können die dem Fachmann für diese Umsetzung bekannten Katalysatoren und Reaktoren (z.B. Festbett- oder Suspensionsfahrweise) sowie Verfahren (kontinuierlich, halbkontinuierlich, diskontinuierlich) angewendet werden.

Bei der Katalysatorfestbettfahrweise ist sowohl die Sumpf- als auch die Rieselfahrweise möglich. Bevorzugt ist eine Rieselfahrweise.

Diesbezüglich wird hiermit z.B. auf die in den Anmeldungen GB-A-852,972 (Äquivalent: DE-A-11 19 285) (BASF AG), DE-A-12 59 899 (BASF AG) und dem US Patent Nr. 3,069,469 (California Research Corp.) beschriebenen Verfahren verwiesen.

Der Hydrierreaktor kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes.

Damit lässt sich eine optimale Verdünnung der Reaktionslösung erreichen, was sich günstig auf die Selektivität auswirkt. Insbesondere kann der Kreislaufstrom mittels eines externen Wärmeüberträgers auf einfache und kostengünstige Weise gekühlt und somit die Reaktionswärme abgeführt werden. Der Reaktor lässt sich dadurch auch adiabat betreiben, wobei der Temperaturanstieg der Reaktionslösung durch den gekühlten Kreislaufstrom begrenzt werden kann. Da der Reaktor selbst dann nicht gekühlt werden muss, ist eine einfache und kostengünstige Bauform möglich. Eine Alternative stellt ein gekühlter Rohrbündelreaktor dar.

Bevorzugt sind Katalysatoren, die Kobalt und/oder Nickel und/oder Eisen, als Vollkatalysator oder auf einem inerten Träger, enthalten.

Hierbei liegen die Reaktionstemperaturen im allgemeinen bei 40 bis 150°C, bevorzugt bei 40 bis 120°C.

Der Druck liegt im allgemeinen bei 40 bis 300 bar, bevorzugt 100 bis 200 bar.

### Isolierung des MXDAs:

Nach der Hydrierung werden das Lösungsmittel und der gegebenenfalls eingesetzte Ammoniak abdestilliert.

Bevorzugt erfolgt eine Reinigung des Xylylendiamins durch Abdestillation leichtersiedender Nebenprodukte (bei gleichem Druck) über Kopf und destillativer Abtrennung von schwerersiedenden Verunreinigungen über Sumpf.

Besonders bevorzugt ist die Fahrweise, in der man nach der Hydrierung das NMP, gegebenenfalls Ammoniak sowie gegebenenfalls leichtersiedende Nebenprodukte über Kopf abdestilliert und danach schwerersiedende Verunreinigungen vom meta-Xylylendiamin destillativ über Sumpf abtrennt.

In einer besonderen Ausführungsform kann die Abtrennung leichter- und schwerersiedender Nebenprodukte auch in einer Seitenabzugs- oder Trennwandkolonne erfolgen, wobei reines meta-Xylylendiamin über einen flüssigen oder gasförmigen Seitenabzug gewonnen wird.

Je nach gewünschter Reinheit wird das Produkt (MXDA) zusätzlich mit einem organischen Lösungsmittel, bevorzugt einem aliphatischen Kohlenwasserstoff, insbesondere einem cycloaliphatischen Kohlenwasserstoff, ganz besonders Cyclohexan oder Methylcyclohexan, extrahiert.
Diese Reinigung durch Extraktion kann z.B. gemäß DE-A-1 074 592 erfolgen.

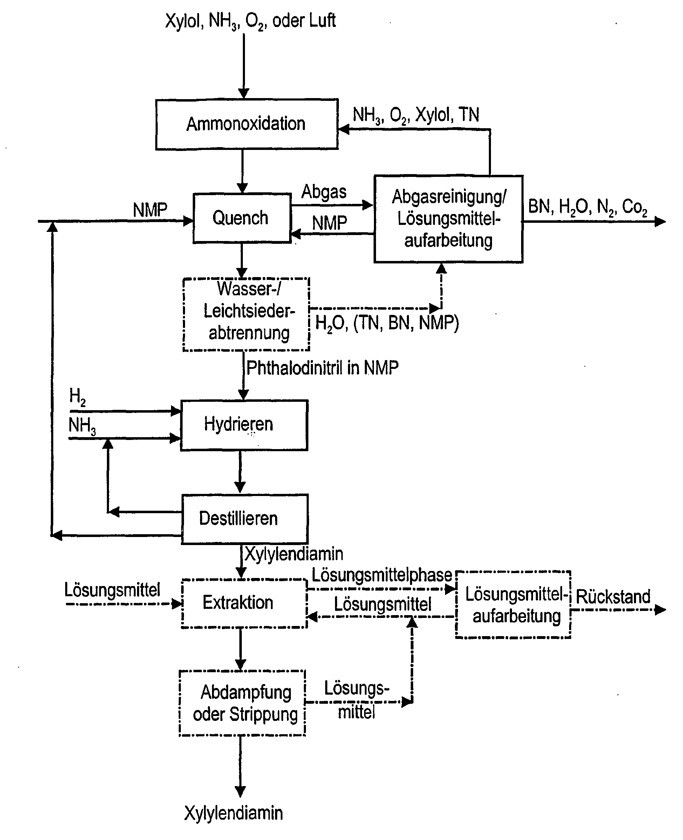

Einen schematischen Überblick über eine bevorzugte Ausgestaltung des erfindungsgemäßen Verfahrens gibt die Abbildung 1 in der Anlage.
Die optionalen Verfahrensschritte ,Wasserabtrennung' und ,extraktive XDA-Reinigung sind gestrichelt gezeichnet.

### Beispiele

### Beispiel 1:

Ammonoxidation von m-Xylol, anschließendes Quenchen der Reaktionsgase mit NMP als Lösungsmittel und Hydrierung des in der Ammonoxidationsstufe entstandenen IPDNs (vergl. Verfahrensschema in Abbildung 1)

Ein Katalysator der Zusammensetzung V₄Sb₃W_{0,4}Cs_{0,2} auf Steatit wurde als Festbett in einen Rohrreaktor eingebaut. Die Apparatur wurde von außen auf 400°C aufgeheizt. Dem Reaktor wurde verdampftes m-Xylol, gasförmiger Ammoniak, Luft und Stickstoff zugefahren (NH₃ / m-Xylol = 8 mol / 1 mol; O₂ / m-Xylol = 4 mol / 1 mol). Der vordere Teil des Reaktors war mit einer Inertschüttung gefüllt, so dass die Einsatzstoffe vorgemischt und auf 400°C vorgeheizt die Reaktionszone erreichten. Im Reaktor herrschte ein leichter Überdruck von 0,02-0,03 bar. Die Hot-Spot-Temperatur erreichte 450°C.

Man erhielt bei einem Umsatz (U) von m-Xylol von 79 % eine Selektivität (S) zu IPDN von 68 %.

Das aus dem Reaktor austretende Gasgemisch wird in einer Kolonne mit NMP gequencht. Aus der Quenchkolonne wird bei 122°C eine Lösung von IPDN in NMP ausgetragen, welche 0,6 Gew.-% m-Xylol, 1,6 Gew.-% Wasser, 0,1 Gew.-% Benzonitril, 4 Gew.-% Tolunitril, 27 Gew.-% IPDN und ca. 66,7 Gew.-% NMP enthält. Über Kopf der Quenchkolonne werden nicht umgesetzte Reaktionsgase und Inertgase sowie nicht umgesetztes m-Xylol sowie etwas NMP gasförmig abgezogen. Dieses Gas kann aufgearbeitet werden, um die Wertstoffe (insbesondere NH₃, m-Xylol, NMP sowie Tolunitril) in die Reaktionsstufe bzw. in den Quenchkreis zurückzuführen. Inerte und Begleitkomponenten (H₂O, Benzonitril, N₂, CO₂, etc.) werden aus der Aufarbeitungsstufe ausgeschleust.

Eine Lösung entsprechend der berechneten Zusammensetzung am Quenchaustritt, bestehend aus 0,44 g m-Xylol, 1,7 g Wasser, 0,88 g Benzonitril, 3,1 g Tolunitril, 24 g IPDN und 58 g NMP, wurde aus den reinen Komponenten zusammengemischt und der Hydrierung zugeführt. Zur Hydrierung wurde der Mischung flüssiges NH₃ zudosiert (NH₃ / IPDN = 14 mol / 1 mol). Die Hydrierung erfolgte in Gegenwart von H₂ und 5 g Raney-Nickel-Katalysator bei 100°C und einem Druck von 100 bar in einem Rührautoklaven.
Die Umsetzung von IPDN war quantitativ, wobei eine Ausbeute von 92 % bezogen auf eingesetztes IPDN erhalten wurde.

(Die oben angegebenen Daten des Quenchschrittes sind die Ergebnisse einer thermodynamischen Simulation. Dabei wurde der Quench als Apparat gerechnet, in dem thermodynamisches Gleichgewicht zwischen Gas- und Flüssigphase herrscht. Neben den Reinstoffdaten der beteiligten Komponenten wurden bei der Berechnung reale Binärdaten verwendet. Derartige Berechnungen können mit kommerziellen Rechenprogrammen, hier: Aspen Plus, die dem Fachmann geläufig sind, durchgeführt werden).

### Beispiel 2:

Die Ammonoxidation wurde wie im Beispiel 1 durchgeführt, jedoch beträgt die Austrittstemperatur aus dem Quench 91°C. Der Wassergehalt der Lösung beträgt 5,2 Gew.-%. Die Lösung wird einer Kolonne mit 15 Glockenböden zugefahren. Der Zulauf ist auf dem 6. Glockenboden. Das Wasser wird zusammen mit anderen Komponenten (gelöste Gase, m-Xylol, Benzonitril, Tolunitril, letztere als Azeotrope mit Wasser) über Kopf abgetrennt. Über Sumpf wird eine praktisch wasserfreie Lösung bestehend aus ca. 27 Gew.% IPDN, 67 Gew.-% NMP, 6 Gew.-% Tolunitril und 600 Gew.-ppm Benzonitril erhalten.

Eine Mischung bestehend aus 27 Gew.-% IPDN und 73 Gew.-% NMP, die aus den reinen Komponenten zusammengemischt wurde, wurde in einem kontinuierlich betriebenen 70 ml-Rohrreaktor an einem Kobalt-Vollkontakt bei 80°C und 190 bar hydriert. Über den Katalysator wurden stündlich 70 g IPDN-Lösung sowie 90 g Ammoniak geleitet. Die Ausbeute an MXDA betrug 96 % bezogen auf eingesetztes IPDN.

(Die oben angegebenen Daten des Quenchschrittes sind die Ergebnisse einer thermodynamischen Simulation, durchgeführt wie bei Beispiel 1).

### Beispiel 3:

Eine Mischung bestehend aus 27 Gew.% IPDN und 73 Gew.-% NMP, die aus den reinen Komponenten zusammengemischt wurde, wurde in einem kontinuierlich betriebenen 70 ml-Rohrreaktor an einem Kobalt-Vollkontakt bei 80°C und 190 bar hydriert. Über den Katalysator wurden stündlich 70 g IPDN-Lösung sowie 54 g Ammoniak geleitet. Die gleiche Volumenmenge wurde als Lösemittel im Kreis gefahren. Die Ausbeute an MXDA betrug 95,5 % bezogen auf eingesetztes IPDN.

### Beispiel 4:

Eine Mischung bestehend aus 15 Gew.-% IPDN und 85 Gew.-% NMP, die aus den reinen Komponenten zusammengemischt wurde, wurde in einem kontinuierlich betriebenen 70 ml-Rohrreaktor an einem Kobalt-Vollkontakt bei 80°C und 190 bar hydriert. Über den Katalysator wurden stündlich 140 g IPDN-Lösung sowie 72 g Ammoniak geleitet. Die Ausbeute an MXDA betrug 96 % bezogen auf eingesetztes IPDN.

### Beispiel 5:

Ammonoxidation von m-Xylol, anschließendes Quenchen der Reaktionsgase mit NMP als Lösungsmittel

Ein Katalysator der Zusammensetzung V₄Sb₃K_{0,4}Ba_{0,2} auf Steatit wurde als Festbett in einen Rohrreaktor eingebaut. Die Apparatur wurde von außen auf 415°C aufgeheizt. Dem Reaktor wurde verdampftes m-Xylol, gasförmiger Ammoniak und Luft zugefahren (NH₃ / m-Xylol =14 mol / 1 mol; O₂ / m-Xylol = 4 mol / 1 mol). Der Katalysator der ersten Hälfte des Reaktors war mit 70 Gew.-% Steatitkugeln verdünnt, die zweite Hälfte mit 40 Gew.-%. Im Reaktor herrschte ein leichter Überdruck von 0,02 bar. Die Hot-Spot-Temperatur erreichte 430°C. Man erhielt bei einem Umsatz von m-Xylol von 88 % eine Selektivität zu IPDN von 71 %.

Das aus dem Reaktor austretende Gasgemisch wird in einer Kolonne mit NMP gequencht. Aus der Quenchkolonne wird bei 120°C und 1,02 bar (abs.) eine Lösung von IPDN in NMP ausgetragen, welche 0,25 Gew.-% m-Xylol, 1,3 Gew.-% Wasser, 3,6 Gew.-% Tolunitril, 27 Gew.-% IPDN und ca. 67,7 Gew.-% NMP enthält. Über Kopf der Quenchkolonne werden nicht umgesetzte Reaktionsgase und Inertgase sowie nicht umgesetztes m-Xylol sowie etwas NMP gasförmig abgezogen. Dieses Gas kann aufgearbeitet werden, um die Wertstoffe (insbesondere NH₃, m-Xylol, NMP sowie Tolunitril) in die Reaktionsstufe bzw. in den Quenchkreis zurückzuführen. Inerte und Begleitkomponenten (H₂O, N₂, CO₂, etc.) werden aus der Aufarbeitungsstufe ausgeschleust.

(Die oben angegebenen Daten des Quenchschrittes sind die Ergebnisse einer thermodynamischen Simulation, durchgeführt wie bei Beispiel 1).

### Beispiel 6:

Untersuchungen zur Löslichkeit von IPDN in verschiedenen Lösungsmitteln

Die Löslichkeit von IPDN in NMP beträgt bei 60°C ca. 26 Gew.-% und bei 90°C ca. 41 Gew.-%.
Pseudocumol erreicht bei 90°C lediglich eine Löslichkeit von 20 Gew.-% und Mesitylen lediglich von 12 Gew.-%.
Bei 60°C liegt die Löslichkeit von IPDN in Mesitylen oder Pseudocumol jeweils unter 10 Gew.-%.

## Patentansprüche

1. Verfahren zur Herstellung von meta-Xylylendiamin umfassend die Schritte Ammonoxidation von meta-Xylol zu Isophthalodinitril, wobei das dampfförmige Produkt der Ammonoxidationsstufe direkt mit einem flüssigen organischen Lösungsmittel in Kontakt gebracht wird (Quench) und
Hydrierung des lsophthalodinitrils in der erhaltenen Quenchlösung oder -suspension,
**dadurch gekennzeichnet, dass** es sich bei dem organischen Lösungsmittel um N-Methyl-2-pyrrolidon (NMP) handelt.

2. Verfahren nach Anspruch 1, **dadurch gekennzeichnet, dass** vor der Hydrierung des Isophthalodinitrils aus der erhaltenen Quenchlösung oder -suspension Wasser und gegebenenfalls Produkte mit einem Siedepunkt niedriger als Phthalodinitril (Leichtsieder) teilweise oder vollständig destillativ abgetrennt werden.

3. Verfahren nach einem der vorhergehenden Ansprüche, **dadurch gekennzeichnet, dass** vor der Hydrierung des Isophthalodinitrils keine Abtrennung von Produkten mit einem Siedepunkt höher als Isophthalodinitril (Hochsiedern) aus der erhaltenen Quenchlösung oder -suspension erfolgt.

4. Verfahren nach einem der vorhergehenden Ansprüche, **dadurch gekennzeichnet, dass** die Ammonoxidation bei Temperaturen von 300 bis 500°C an einem Katalysator enthaltend V, Sb und/oder Cr, als Vollkatalysator oder auf einem inerten Träger, durchgeführt wird.

5. Verfahren nach einem der vorhergehenden Ansprüche, **dadurch gekennzeichnet, dass** bei dem Quench mit NMP die Temperatur des Quenchaustrags 40 bis 180°C beträgt.

6. Verfahren nach einem der vorhergehenden Ansprüche, **dadurch gekennzeichnet, dass** die Hydrierung in Gegenwart von Ammoniak durchgeführt wird.

7. Verfahren nach einem der vorhergehenden Ansprüche, **dadurch gekennzeichnet, dass** die Hydrierung bei Temperaturen von 40 bis 150°C an einem Katalysator enthaltend Ni, Co und/oder Fe, als Vollkatalysator oder auf einem inerten Träger, durchgeführt wird.

8. Verfahren nach einem der vorhergehenden Ansprüche, **dadurch gekennzeichnet, dass** nach der Hydrierung eine Reinigung des meta-Xylylendiamins durch Abdestillation von NMP, gegebenenfalls Ammoniak sowie gegebenenfalls leichtersiedender Nebenprodukte über Kopf und destillativer Abtrennung von schwerersiedenden Verunreinigungen über Sumpf erfolgt.

9. Verfahren nach einem der vorhergehenden Ansprüche, **dadurch gekennzeichnet, dass** man nach der Hydrierung das NMP, gegebenenfalls Ammoniak sowie gegebenenfalls leichtersiedende Nebenprodukte über Kopf abdestilliert und danach schwerersiedende Verunreinigungen vom meta-Xylylendiamin destillativ über Sumpf abtrennt.

10. Verfahren nach einem der beiden vorhergehenden Ansprüche, **dadurch gekennzeichnet, dass** das meta-Xylylendiamin nach der Destillation zur weiteren Reinigung mit einem organischem Lösungsmittel extrahiert wird.

11. Verfahren nach dem vorhergehenden Anspruch, **dadurch gekennzeichnet, dass** man zur Extraktion Cyclohexan oder Methylcyclohexan verwendet.

## Claims

1. A process for preparing meta-xylylenediamine, comprising the steps of ammoxidizing meta-xylene to isophthalonitrile, by contacting the vaporous product of the ammoxidation stage directly with a liquid organic solvent (quench), and hydrogenating the isophthalonitrile in the resulting quench solution or suspension, wherein the organic solvent is N-methyl-2-pyrrolidone (NMP).

2. The process according to claim 1, wherein, before the hydrogenation of the isophthalonitrile, water and any products having a boiling point lower than phthalonitrile (low boilers) are partly or fully removed by distillation from the resulting quench solution or suspension.

3. The process according to any of the preceding claims, wherein, before the hydrogenation of the isophthalonitrile, there is no removal of products having a boiling point higher than isophthalonitrile (high boilers) from the resulting quench solution or suspension.

4. The process according to any of the preceding claims, wherein the ammoxidation is carried out at temperatures of from 300 to 500°C over a catalyst comprising V, Sb and/or Cr, as an unsupported catalyst or on an inert support.

5. The process according to any of the preceding claims, wherein the temperature of the quench effluent in the quench with NMP is from 40 to 180°C.

6. The process according to any of the preceding claims, wherein the hydrogenation is carried out in the presence of ammonia.

7. The process according to any of the preceding claims, wherein the hydrogenation is carried out at temperatures of from 40 to 150°C over a catalyst comprising Ni, Co and/or Fe, as an unsupported catalyst or on an inert support.

8. The process according to any of the preceding claims, wherein, after the hydrogenation, the meta-xylylenediamine is purified by distilling off NMP, any ammonia, and also any relatively low-boiling by-products, via the top and distillatively removing relatively high-boiling impurities via the bottom.

9. The process according to any of the preceding claims, wherein, after the hydrogenation, the NMP, any ammonia, and also any relatively low-boiling by-products, are distilled off via the top and, afterwards, any relatively high-boiling impurities are removed from the meta-xylylenediamine by distillation via the bottom.

10. The process according to either of the two preceding claims, wherein the meta-xylylenediamine, after the distillation, is extracted for further purification with an organic solvent.

11. The process according to the preceding claim, wherein cyclohexane or methylcyclohexane are used for the extraction.

## Revendications

1. Procédé de préparation de méta-xylylènediamine, comprenant les étapes d'ammoxydation de méta-xylène en dinitrile isophtalique, le produit obtenu sous forme de vapeur de l'étape d'ammoxydation étant directement mis en contact avec un solvant organique liquide (refroidissement rapide), et d'hydrogénation du dinitrile isophtalique dans la solution ou la suspension de refroidissement rapide obtenue, **caractérisé en ce que** le solvant organique est de la N-méthyl-2-pyrrolidone (NMP).

2. Procédé suivant la revendication 1, **caractérisé en ce que**, avant l'hydrogénation du dinitrile isophtalique de la solution ou suspension de refroidissement rapide obtenue, on sépare par distillation, en tout ou en partie, de l'eau et le cas échéant des produits ayant un point d'ébullition inférieur à celui du dinitrile phtalique (composants à bas point d'ébullition).

3. Procédé suivant l'une quelconque des revendications qui précèdent, **caractérisé en ce que**, avant l'hydrogénation du dinitrile isophtalique, on n'opère pas de séparation de produits ayant un point d'ébullition supérieur à celui du dinitrile isophtalique (composants à haut point d'ébullition) de la solution ou suspension de refroidissement rapide obtenue.

4. Procédé suivant l'une quelconque des revendications qui précèdent, **caractérisé en ce que** l'ammoxydation est entreprise à des températures de 300 à 500°C sur un catalyseur contenant du V, du Sb et/ou du Cr, sous forme de catalyseur plein ou sur un support inerte.

5. Procédé suivant l'une quelconque des revendications qui précèdent, **caractérisé en ce que**, lors du refroidissement rapide avec de la NMP, la température de sortie du refroidissement rapide est de 40 à 180°C.

6. Procédé suivant l'une quelconque des revendications qui précèdent, **caractérisé en ce que** l'hydrogénation est entreprise en présence d'ammoniac.

7. Procédé suivant l'une quelconque des revendications qui précèdent, **caractérisé en ce que** l'hydrogénation est entreprise à des températures de 40 à 150°C sur un catalyseur contenant du Ni, du Co et/ou du Fe, sous forme de catalyseur plein ou sur un support inerte.

8. Procédé suivant l'une quelconque des revendications qui précèdent, **caractérisé en ce que**, après l'hydrogénation, on opère une purification de la méta-xylylènediamine en éliminant par distillation de la NMP, le cas échéant de l'ammoniac et le cas échéant des sous-produits à plus bas point d'ébullition en tête de colonne, et en éliminant par distillation, en fond de colonne, des impuretés à plus haut point d'ébullition.

9. Procédé suivant l'une quelconque des revendications qui précèdent, **caractérisé en ce que**, après l'hydrogénation, on sépare par distillation la NMP, le cas échéant de l'ammoniac et le cas échéant des sous-produits à plus bas point d'ébullition en tête de colonne, et on sépare ensuite des impuretés à plus haut point d'ébullition de la méta-xylylènediamine, par distillation, en fond de colonne.

10. Procédé suivant l'une quelconque des revendications qui précèdent, **caractérisé en ce que** la méta-xylylènediamine est extraite, après la distillation et au moyen d'un solvant organique, aux fins de la purification ultérieure.

11. Procédé suivant la revendication qui précède, **caractérisé en ce que** l'on utilise pour l'extraction du cyclohexane ou du méthylcyclohexane.
